# EUROPEAN PATENT APPLICATION

(11) **EP 4 344 666 A1**
(43) Date of publication of application: **03.04.2024**
(21) Application number: 23199410.4
(22) Date of filing: 25.09.2023
(51) Int. Cl.: A61B 34/00, A61B 34/35

(54) **REMOTE SURGERY SUPPORT SYSTEM**

(30) Priority: 28.09.2022 JP 2022155134
(71) Applicant: Medicaroid Corporation, Kobe-shi, Hyogo 650-0047 (JP)
(72) Inventor: OHASHI, Masanao, Hyogo, 650-0047 (JP); KITATSUJI, Hiroaki, Hyogo, 650-0047 (JP)
(74) Representative: Müller Hoffmann & Partner

(57) **Abstract**

An embodiment of a remote surgery support system includes a surgical system placed in a first facility and a mentor-side operating device placed in a second facility different from the first facility, the surgical system including first and second manipulator arms holding first and second surgical instruments, a third manipulator arm holding an endoscope, and a doctor-side operating device that includes a first display to display an endoscopic image acquired by the endoscope. The mentor-side operating device is configured to operate, via an external network from the second facility, the first to third manipulator arms of the surgical system in the first facility. The mentor-side operating device includes a second display configured to display the endoscopic image transmitted through the external network with the endoscopic image being superimposed with a graphical user interface including information about a state of communication between the mentor-side operating device and the surgical system.

## Description

### BACKGROUND

The disclosure may relate to a remote surgery support system and may particularly relate to a remote surgery support system that is capable of performing surgery under a guidance of a mentor.

In a related art, there has been known a robot system that allows surgery while a surgeon is being mentored by a mentor (a guidance doctor, an experienced doctor, a supervising doctor, a preceptor, a proctor, or the like). (See, for example, Patent Document 1)

Patent Document 1 discloses a robotic system comprising a surgeon master control station, a mentor master control station, and a surgical robot to be operated by the surgeon master control station and the mentor master control station. In the robot system, surgery is performed while the mentor mentors the surgeon through voice communication between the surgeon and the mentor using headsets between the surgeon master control station and the mentor master control station. Also, in this robotic system, the surgeon master control station and the mentor master control station are located at the same facility where the surgical robot is located.

Patent Document 1: Japanese Patent Application Publication No. 2007-181670

### SUMMARY

In the robot system described in Patent Document 1, the surgeon master control station and the mentor master control station are located in the same facility where the surgical robot is located. For this reason, it is difficult for the mentor to provide surgical guidance without going to the facility where the surgery is being performed. In other words, it is difficult for the mentor to provide surgical guidance using the robot system if the mentor is remotely located. If an operating device to be operated by the mentor and the surgical robot were connected via a network in order for the mentor to remotely instruct the surgical operation using the robot system, the operation of the surgical robot would be affected by a state of communication between the operating device of the mentor and the surgical robot, such as a delay. Thus, it may be difficult for the mentor to smoothly operate the surgical robot due to such a delay caused by the communication state.

An object of an embodiment of the disclosure is to provide a remote surgery support system that allows a mentor to grasp a state of communication between a mentor-side operating device and a surgical robot and to perform surgery remotely from the mentor-side operating device according to the communication state.

A first aspect of the disclosure may be a remote surgery support system that may include: a surgical system located at a first facility and including a first manipulator arm holding a first surgical instrument, a second manipulator arm holding a second surgical instrument, a third manipulator arm holding an endoscope, and a doctor-side operating device configured to be operated by a doctor to operate the first to third manipulator arms; a mentor-side operating device that is located in a second facility different from the first facility and configured to be operated by a mentor to operate via an external network the first to third manipulator arms; and one or more controllers. The doctor-side operating device includes a first display configured to display an endoscopic image acquired by the endoscope. The mentor-side operating device includes a second display configured to display the endoscopic image transmitted via the external network. The one or more controllers is configured to acquire a state of communication between the mentor-side operating device and the surgical robot and display, on the second display, a graphical user interface including information about the communication state with the graphical user interface being superimposed with the endoscopic image.

According to the first aspect described above, the one or more controllers acquires the state of communication between the mentor-side operating device and the surgical robot, and displays, on the second display, the graphical user interface including the information about the communication state with the graphical user interface being superimposed with the endoscopic image. With this configuration, even if an operation delay occurs due to the state of communication between the mentor-side operating device and the surgical robot that are remotely connected via the external network, the graphical user interface including the information about the communication state that is displayed on the second display allows the mentor to easily ascertain the communication state. As a result, while grasping the communication state between the mentor-side operating device and the surgical robot, the mentor can remotely operate the surgical robot from the mentor-side operating device according to the communication state.

A second aspect of the disclosure may be a mentor-side operating device. The mentor-side operating device is placed in a second facility different from a first facility in which a surgical system is placed, the surgical system of the first facility including a first manipulator arm holding a first surgical instrument of a surgical robot; a second manipulator arm holding a second surgical instrument of the surgical robot; a third manipulator arm holding an endoscope of the surgical robot; and a doctor-side operating device that includes a first display configured to display an endoscopic image acquired by the endoscope. The mentor-side operating device is configured to operate, via an external network from the second facility, the first to third manipulator arms of the surgical system in the first facility. The mentor-side operating device includes: a second display configured to display the endoscopic image acquired by the endoscope and transmitted through the external network, wherein the second display is configured to display the endoscopic image superimposed with a graphical user interface including information about a state of communication between the mentor-side operating device and the surgical robot.

According to the second aspect of the disclosure as described above, the second display displays the endoscopic image superimposed with the graphical user interface containing the information about the state of communication between the mentor-side operating device and the surgical robot. With this configuration, even if an operation delay occurs due to the state of communication between the mentor-side operating device and the surgical robot that are remotely connected via the external network, the graphical user interface including the information about the communication state that is displayed on the second display allows the mentor to easily ascertain the communication state. As a result, while grasping the communication state between the mentor-side operating device and the surgical robot, the mentor can remotely operate the surgical robot from the mentor-side operating device according to the communication state.

According to at least one of the aspects described above, while grasping the state of communication between the mentor-side operating device and the surgical robot, the mentor can remotely operate the surgical robot from the mentor-side operating device according to the communication state.

### BRIEF DESCRIPTION OF DRAWINGS

FIG. 1 is a diagram illustrating a view of a configuration of a remote surgery support system according to an embodiment;
FIG. 2 is a diagram illustrating a view of a surgical robot according to an embodiment;
FIG.3 is a diagram illustrating a view of each of a doctor-side operating device and a mentor-side operating device according to an embodiment;
FIG. 4 is a block diagram illustrating a view of a configuration of the remote surgery support system according to an embodiment;
FIG. 5 is a diagram illustrating a view of a scope type display of the remote surgery support system according to an embodiment;
FIG. 6 is a block diagram illustrating a view of a communication-related configuration of the remote surgery support system according to an embodiment;
FIG. 7 is a diagram illustrating a view of an example of a display screen of the remote surgery support system according to an embodiment;
FIG. 8 is a diagram illustrating a first display example of communication-related information according to an embodiment;
FIG. 9 is a diagram illustrating a second display example of communication-related information according to an embodiment;
FIG. 10 is a diagram illustrating a third display example of communication-related information according to an embodiment;
FIG. 11 is a diagram illustrating a fourth display example of communication-related information according to an embodiment;
FIG. 12 is a diagram illustrating a fifth display example of communication-related information according to an embodiment;
FIG. 13 is a diagram illustrating a sixth display example of communication-related information according to an embodiment;
FIG. 14 is a diagram illustrating a seventh display example of communication-related information according to an embodiment; and
FIG. 15 is a flowchart for explaining a communication status acquisition process according to an embodiment.

### DETAILED DESCRIPTION

Descriptions are provided hereinbelow for one or more embodiments based on the drawings. In the respective drawings referenced herein, the same constituents are designated by the same reference numerals and duplicate explanation concerning the same constituents is omitted. All of the drawings are provided to illustrate the respective examples only.

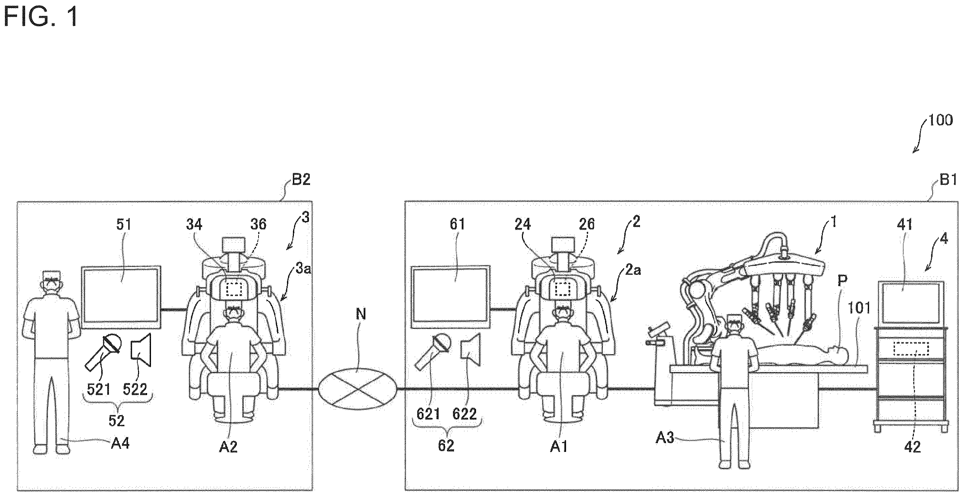

A configuration of a remote surgery support system 100 (a remote surgical system, a telesurgery system) according to an embodiment is described with reference to FIGS. 1 to 15.

As illustrated in FIG. 1, the remote surgery support system 100 includes: a surgical robot 1 placed in the vicinity of an operation table 101 on which a patient P is to be placed; a doctor-side operating device 2 through which a doctor A1 (surgeon A1) operates the surgical robot 1; and a mentor-side operating device 3 through which a mentor A2 (such as a guidance doctor, an experienced doctor, a supervising doctor, a preceptor, a proctor, or the like) operates the surgical robot 1 via an external network N. The surgical robot 1 and the doctor-side operating device 2 are located at a first facility B1. The mentor-side operating device 3 is located in a second facility B2 different from the first facility B1. In the first facility B1, an assistant A3 as a surgical staff is assigned near the surgical robot 1. Further, in the first facility B1, an information sharing device 4 for sharing information with the assistant A3 is placed. In the second facility B2, a clinical engineer A4 is assigned near the mentor-side operating device 3 as a remote surgical staff.

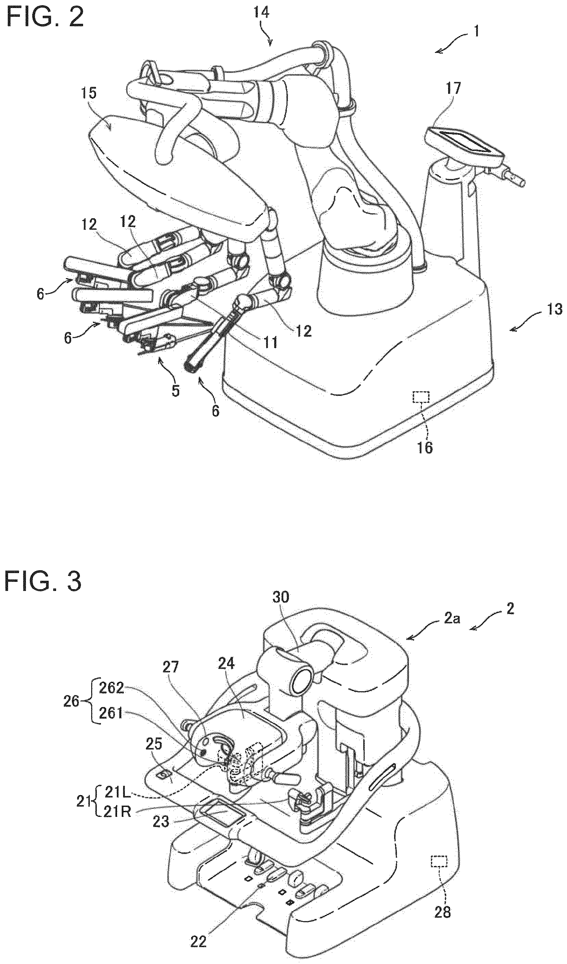

As illustrated in FIG. 2, the surgical robot 1 includes one manipulator arm 11 that holds an endoscope 5 and a plurality (three, in this example) of manipulator arms 12 that respectively hold surgical instruments 6. The endoscope 5 captures an image of a surgical site inside the patient P. An end effector such as forceps or the like is attached to a distal end of each of or at least one of the surgical instruments 6. The doctor-side operating device 2 and the mentor-side operating device 3 are provided for operating the manipulator arms 11 and 12. Note that the left manipulator arm 11 and the right manipulator arm 12 are examples of a "first manipulator arm" and a "second manipulator arm", respectively.

The surgical robot 1 is placed in a surgery room (an operating room) in the first facility B1. The surgical robot 1 includes a medical cart 13, a positioner 14 and an arm base 15. The surgical robot 1 is configured to be movable by the medical cart 13. The medical cart 13 is provided with a controller 16 (or a control device) that controls operation of the surgical robot 1. The controller 16 controls the operation of the surgical robot 1 based on a command input to the doctor-side operating device 2 or the mentor-side operating device 3. The controller 16 includes a processor or a circuitry such as a CPU or the like that executes programs, and a storage such as a memory that stores the programs. Note that the controller 16 is an example of a "controller" or a "control device."

Further, the medical cart 13 is provided with an input device 17. The input device 17 is configured to accept operations to move or change postures of the manipulator arm 11, the manipulator arms 12, the positioner 14, the arm base 15, mainly in order to prepare for surgery before the surgery.

The positioner 14 is configured as a seven-axis articulated robot. The positioner 14 is disposed on the medical cart 13. The positioner 14 is configured to move the arm base 15. Specifically, the positioner 14 is configured to move the position of the arm base 15 three-dimensionally.

The arm base 15 is attached to a distal end of the positioner 14. The manipulator arm 11 and the manipulator arms 12 are attached to the arm base 15 at their base portions (proximal end portions). The arm base 15, the manipulator arm 11, and the manipulator arm 12 are used with being covered with a sterile drape.

As illustrated in FIG. 1, the doctor-side operating device 2 is located, for example, in or outside the surgery room (the operating room) in the first facility B1. The doctor-side operating device 2 includes a doctor-side operating device main body 2a. As illustrated in FIG. 3, the operating device main body 2a includes operating handles 21, foot pedals 22, a touch panel 23, a scope type display 24, an armrest 25, a voice communication device 26 (an audio communication device 26), a sensor 27, a controller 28 (a control device 28), and a support arm 30. The operating handle 21 is an example of a "first operating handle". The touch panel 23 is an example of a "switching device." The scope type display 24 is an example of a "first display" or a "first display device." The voice communication device 26 is an example of the "first voice communication device."

The operating handles 21 are control handles provided for an operator (doctor A1) to input commands. The operating handles 21 are provided for the operator (doctor A1) to operate the manipulator arms 11 and 12 (the endoscope 5 and the surgical instruments 6). The operating handles 21 also receive an amount (an operation amount) to operate the manipulator arms 11 and 12 (the endoscope 5 and the surgical instruments 6). The operating handles 21 comprise a pair of operating handles 21 which include a left-hand operating handle 21L to be operated by the left hand of the operator (doctor A1) and a right-hand operating handle 21R to be operated by the right hand of the operator (doctor A1). The surgical instrument 6 that is held by one manipulator arm 12 is operated by the operating handle 21L, and the surgical instrument 6 that is held by another manipulator arm 12 is operated by the operating handle 21R.

Further, a movement amount of the endoscope 5 or the surgical instrument 6 is scaled (changed) with respect to the operation amount received by the operating handle 21. When the magnification of the movement amount (the scaling ratio) is set to 1/2, for example, the endoscope 5 or the surgical instrument 6 moves 1/2 of the movement distance of the operating handle 21. This allows for precise fine surgery.

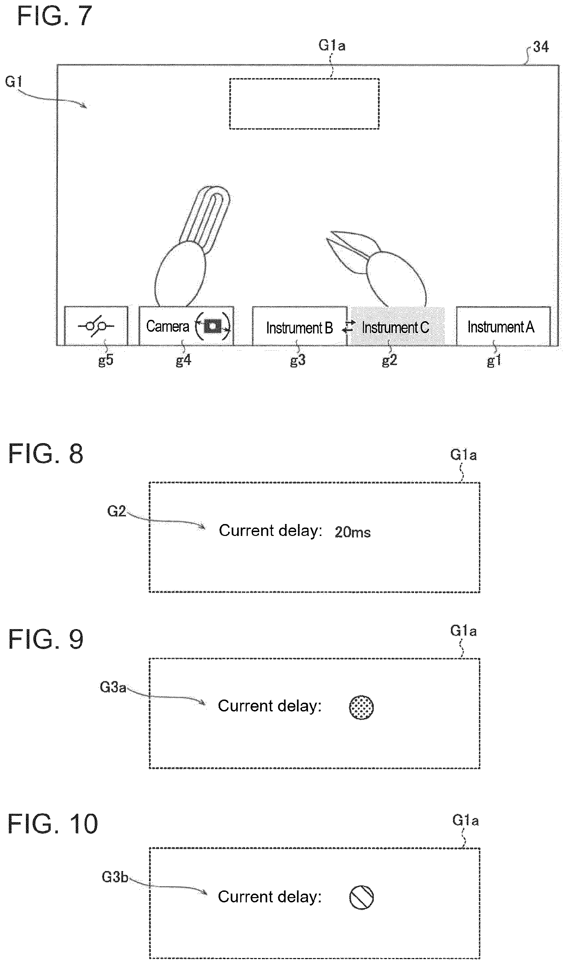

The foot pedals 22 includes a plurality of pedals to perform functions related to the endoscope 5 and the surgical instruments 6. The plural pedals include a coagulation pedal, a cutting pedal, a camera pedal, and a clutch pedal. The plurality pedals are operated by a foot (feet) of the operator (doctor A1). When the camera pedal is operated, the operating handles 21 are allowed to move the endoscope held by the manipulator arm 11. At this time, the endoscope is moved by operating both the operating handle 21L and the operating handle 21R. The touch panel 23 is configured to accept setting operations regarding the doctor-side operating device 2. The touch panel 23 is provided to the armrest 25. The scope type display 24 is configured to display an endoscopic image (see FIG. 7) acquired by the endoscope 5. The scope type display 24 is an immersive display that allows only the operator (doctor A1) to see the displayed image. The operator (doctor A1) operates the operating handles 21 and the foot pedals 22 while viewing a surgical site (or an affected area) displayed on the scope type display 24. The support arm 30 supports the scope type display 24 so that the height of the scope type display 24 can be adjusted to the height of the face of the operator such as a doctor.

The armrest 25 is formed in a shape of a bar, and is configured so that an operator (doctor A1) can put his or her arm thereon when operating the operating handles 21. The voice communication device 26 is provided at a position where the operator (doctor A1) can perform voice communication with a voice communication device 36 (described later) of the mentor-side operating device 3 while looking into the scope type display 24. Specifically, the voice communication device 26 is provided near the head of the operator (doctor A1) when the operator (doctor A1) looks into the scope type display 24. The voice communication device 26 is provided integrally with the scope type display 24 and thus the voice communication device 26 and the scope type display 24 can be integrally adjusted in height by the support arm 30.

The sensor 27 is configured to detect whether or not the scope type display 24 is in a state of use in which the scope type display 24 is looked into. The sensor 27 is provided at a position where the sensor 27 detect the head of the operator (doctor A1) when the operator positions the head thereof to look into the scope type display 24. The sensor 27 is provided integrally with the scope type display 24. The controller 28 controls the operation of the doctor-side operating device 2. The controller 28 includes a processor or a circuitry such as a CPU that executes programs, and a storage such as a memory that stores the programs.

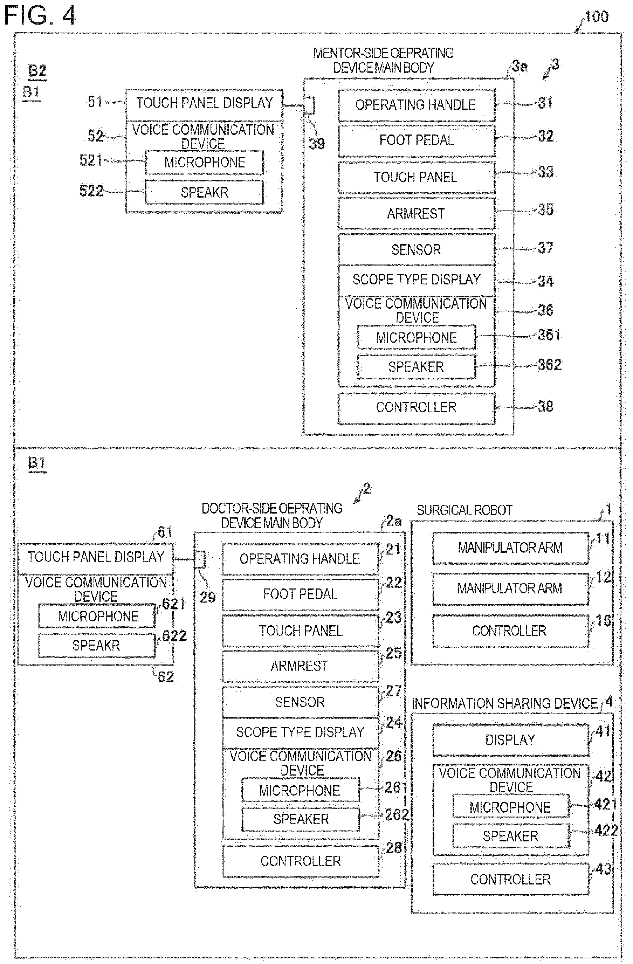

Although the doctor-side operating device 2 has been described above, the mentor-side operating device 3 has the structure same as or similar to the doctor-side operating device 2. That is, as illustrated in FIG. 4, the mentor-side operating device 3 includes a mentor-side operating device main body 3a. The mentor-side operating device main body 3a includes operating handles 31, foot pedals 32, a touch panel 33, a scope type display 34, an armrest 35, the voice communication device 36 (an audio communication device 36), a sensor 37, a controller 38, and a support arm 30. The scope type display 34 is configured to display the endoscopic image (see FIG. 7) transmitted via the external network N (see FIG. 1). Although the doctor-side operating device 2 and the mentor-side operating device 3 are basically the same specifications, the normal mode and the remote mode are switched via the touch panel 23 (33). The operating handle 31 is an example of a "second operating handle". The touch panel 33 is an example of a "switching device." The scope type display 34 is an example of a "second display" or a "second display device." The voice communication device 36 is an example of the "second voice communication device."

As illustrated in FIGS. 1 and 4, the mentor-side operating device 3 includes: a touch panel display 51 configured to display an endoscopic image (see FIG. 7) transmitted via the external network N; and a voice communication device 52 (an audio communication device 52) that is arranged closer to the touch panel display 51 than the scope type display 34 and configured to perform voice communication with the voice communication device 26. Note that the touch panel display 51 is an example of a "third display" or a "third display device." Note that the controller 43 is an example of a "controller" or a "control device." The voice communication device 52 is an example of a "third voice communication device."

The touch panel display 51 and the voice communication device 52, as **parts, units, or devices** independent from the mentor-side operating device main body 3a, are arranged in the vicinity of the mentor-side operating device main body 3a. The touch panel display 51 is, for example, a display having a touch panel function. The touch panel display 51 is a flat panel display or a curved panel display. The mentor A2 causes the touch panel display 51 to receive the instruction input using a finger or a pen for a touch panel to generate an instructional image. The instructional image can be, for example, lines, circles, arrows, and/or the like.

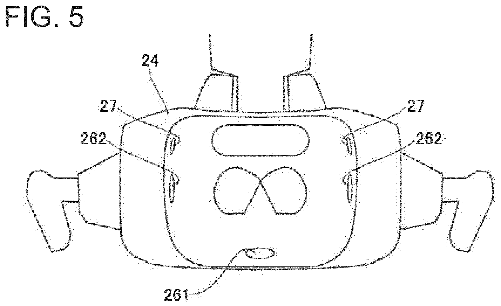

As illustrated in FIGS. 4 and 5, each of the voice communication device 26, the voice communication device 36, and the voice communication device 52 includes a microphone (261, 361, 521) and a speaker (262, 362, 522). The microphone 261 (361, 521) accepts audio input. The speaker 262 (362, 522) outputs audio. As illustrated in FIG. 5, the speaker 262 (362) is provided at a position close to the operator's ear when the operator (doctor A1 or mentor A2) positions his or her head to look into the scope type display 24 (34). The microphone 261 (361) is provided at a position close to the operator's mouth when the operator (doctor A1 or mentor A2) looks into the scope type display 24 (34). The microphone 261 (361) and the speaker 262 (362) are provided integrally with the scope type display 24 (34).

The voice communication device 52 is integrally provided with the touch panel display 51. The voice communication device 52 includes, for example, the microphone 521 and the speaker 522 in the touch panel display 51. Note that In FIG. 1, the touch panel display 51 and the voice communication device 52 are separately illustrated.

Further, the doctor-side operating device 2 includes the units (components) same as in the mentor-side operating device 3. That is, the doctor-side operating device 2 includes: a touch panel display 61 configured to display the endoscopic image G1 (see FIG. 7); and a voice communication device 62 arranged closer to the touch panel display 61 than the scope type display 24 so as to perform voice communication with the voice communication devices 36 and 52. With the touch panel display 61, it is possible to perform the annotation in both directions between the doctor A1 and the mentor A2 during the operation.

The touch panel display 61 and the voice communication device 62, as **parts, units, or devices** independent from the doctor-side operating device main body 2a, are arranged in the vicinity of the doctor-side operating device main body 2a. The touch panel display 61 is, for example, a flat panel display or a curved panel display having a touch panel function.

The voice communication device 62 also includes a microphone 621 and a speaker 622. The microphone 621 accepts voice input. The speaker 622 outputs sound. The voice communication device 62 is integrally provided with the touch panel display 61. The voice communication device 62 includes, for example, the microphone 621 and the speaker 622 in the touch panel display 61. Note that In FIG. 1, the touch panel display 61 and the voice communication device 62 are separately illustrated.

As illustrated in FIGS. 1 and 4, the information sharing device 4 is a movable monitor cart. With reference to FIGS. 1 and 2, the information sharing device 4 includes a display 41 (a display device) configured to display the endoscopic image (see FIG. 7) acquired by the endoscope 5 (see FIG. 2), and a voice communication device 42 (an audio communication device 42) for voice communication with the voice communication device 26 of the doctor-side operating device 2. The information sharing device 4 is located closer to the surgical robot 1 than the doctor-side operating device 2. Therefore, the assistant A3 placed as a surgical staff near the surgical robot 1 can check the endoscopic image through the monitor cart 4 and interact with the doctor A1. The voice communication device 42 is also configured to perform voice communication with the voice communication device 36 and the voice communication device 52. This allows the mentor A2 and the clinical engineer A4 to hear the dialogue between the doctor A1 and the assistant A3 as a surgical staff assigned near the surgical robot 1. The assistant A3 can also interact with the mentor A2 and the clinical engineer A4. The voice communication device 42 also includes a microphone 421 and a speaker 422. The microphone 421 accepts voice input. The speaker 422 outputs sound. The display 41 is, for example, a flat panel display or a curved panel display.

Further, a controller 43 is disposed in the information sharing device 4. The controller 43 performs image processing. The controller 43 includes a processor or a circuitry such as a CPU that executes programs, and a storage such as a memory that stores the programs. Further, the controller 43 is arranged at the first facility B1.

Note that the controller 43 displays the same image on the scope type display 24, the scope type display 34, the display 41, the touch panel display 51, and the touch panel display 61. That is, the same endoscopic image is displayed on the scope type display 24, the scope type display 34, the display 41, the touch panel display 51, and the touch panel display 61.

As illustrated in FIGS. 3 to 5, the mentor-side operating device 3 includes the sensor 37 configured to detect whether or not the mentor-side operating device 3 is in the state of use in which the scope type display 34 is looked into, as described above. The controller 16 is configured, when the sensor 37 does not detect the state of use, to prohibit the manipulator arms 11 and 12 from being operated by the operating handles 31, and when the sensor 37 detects the state of use, to permit the manipulator arms 11 and 12 to be operated by the operating handles 31. The controller 16 is configured to, in a state where the sensor 37 does not detect the state of use, prohibit the operations of the manipulator arms 11 and 12 by not accepting input from the operating handles 31. To the contrary, the controller 16 is configured to, in a state where the sensor 37 detects the state of use, accept predetermined inputs from the operating handles 31 by operably connecting the manipulator arms 12 to the operating handles 31.

Note that the same applies to the doctor-side operating device 2. That is, the controller 16 is configured, when the sensor 27 does not detect the state of use, to prohibit the manipulator arms 11 and 12 from being operated by the operating handles 21, and configured, when the sensor 27 detects the state of use, to operably connecting the manipulator arms 11 and 12 to the operating handles 21 so as to accept predetermined inputs from the operating handles 21 to operate the manipulator arms 11 and 12.

Each of the doctor-side operating device 2 and the mentor-side operating device 3 is provided with a switching device configured to switch an operation authority (operation permission) for operating the surgical robot 1 between the doctor-side operating device 2 and the mentor-side operating device 3. The switching device is arranged on each of the armrests 25 and 35. The switching device is the touch panel 23 (33) disposed on the armrest 25 (35). Thus, by operating the touch panel 23 (33) provided on the armrest 25 (35), the operation of the surgical robot 1 can be switched more easily between the doctor-side operating device 2 and the mentor-side operating device 3. Also, the switching device may be provided as a switch on the operating handle 21 (31). Accordingly, the operation of the surgical robot 1 can be more easily switched between the doctor-side operating device 2 and the mentor-side operating device 3 by the switch provided on the operating handle 21 or the operating handle 31.

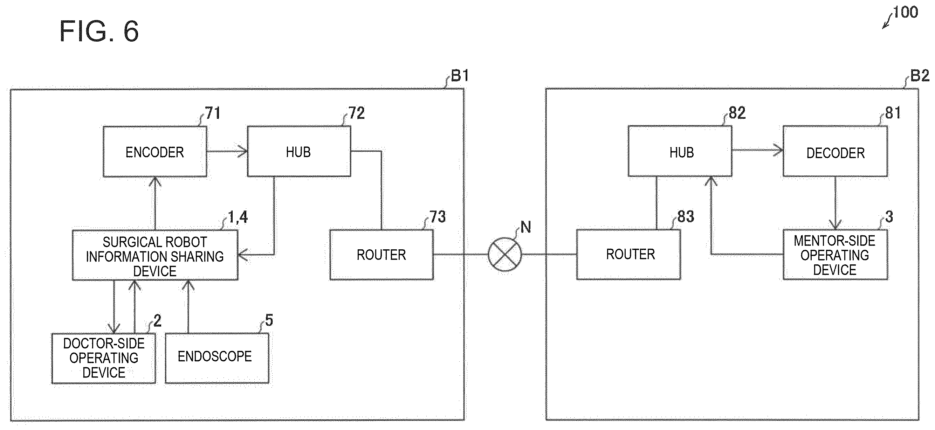

As illustrated in FIG. 6, the surgical robot 1, the doctor-side operating device 2, and the information sharing device 4 provided at the first facility B1 are connected via a network N to the mentor-side operating device 3 provided at the second facility B2. Specifically, the first facility B1 is provided with an encoder 71 configured to convert the image generated by the information sharing device 4 into transmittable data, a hub 72, and a router 73. The second facility B2 is also provided with a decoder 81 configured to restore the transmitted data to the image, a hub 82, and a router 83.

The image acquired by the endoscope 5 is input to the information sharing device 4. The information sharing device 4 is configured to generate a graphical user interface G1 including display areas g1, g2, g3, g4 and g5 as illustrated in FIG. 7 and superimposes the graphical user interface G1 on the endoscopic image. The display area g1 displays information on the surgical instrument 6 being operated with the operating handle 21R, the display area g2 displays information on the surgical instrument 6 for replacement, and the display area g3 displays information on the surgical instrument 6 being operated with the operating handle 21L, the display area g4 displays information of operation of the camera pedal, and the display area g5 displays information of operation of the clutch pedal. The endoscopic image on which the graphical user interface G1 is superimposed **by the information sharing device 4** is displayed on the display 41 and is transmitted to the doctor-side operating device 2 and displayed on the scope type display 24 of the doctor-side operating device 2. Also, the endoscopic image on which the graphical user interface G1 generated by the information sharing device 4 is superimposed is sent to the encoder 71 and converted into transmittable data. The data converted by encoder 71 is sent through the hub 72 and the router 73 to the second facility B2 via the external network N. At the second facility B2, the converted data is sent to the decoder 81 via the router 83 and the hub 82. The decoder 81 restores the received data into the endoscopic image superimposed with the graphical user interface G1, which is sent to the mentor-side operating device 3 and displayed on the scope type display 34.

Signals generated based on the operation on the doctor-side operating device 2 of the first facility B1 is sent to the surgical robot 1, so that the surgical instruments 6 and the endoscope 5 of the surgical robot 1 are operated.

Signals generated based on the operation on the mentor-side operating device 3 of the second facility B2 is sent to the surgical robot 1 via the hub 82, the router 83, the external network N, the router 73, and the hub 72, so that the surgical instruments 6 and endoscope 5 of the surgical robot 1 are operated.

In addition, in voice communication between the doctor-side operating device 2, the information sharing device 4 and the voice communication device 62 installed at the first facility B1 and the mentor-side operating device 3 and the voice communication device 52 installed at the second facility B2, information is transmitted and received via the hub 72, the router 73, the external network N, the router 83 and the hub 82.

In an embodiment, the controller 16 acquires the state of communication between the mentor-side operating device 3 and the surgical robot 1. Then, as illustrated in FIGS. 7 to 14, the controller 43 generates the graphical user interface G1 including information about the state of communication acquired by the controller 16, superimposes the graphical user interface G1 with the endoscopic image, and displays on the scope type display. 34 the graphical user interface G1 superposed with the endoscopic image.

As a result, even if an operation delay occurs due to the state of communication between the mentor-side operating device 3 and the surgical robot 1 that are remotely connected via the external network N, the graphical user interface G1 including the information about the communication status displayed on the scope type display 34 allows the mentor A2 to easily check the communication state. As a result, while grasping the communication state between the mentor-side operating device 3 and the surgical robot 1, the mentor A2 can remotely operate the surgical robot 1 from the mentor-side operating device 3 with reference to the communication state. For example, if there is a delay in operation, the mentor A2 can recognize how much delay has occurred through the graphical user interface G1 and operate the surgical robot 1 while taking the degrees of the delay into consideration.

The controller 43 is arranged at the first facility B1, and displays the endoscopic image superimposed with the graphical user interface G1 on the scope type display 34 via the external network N. As a result, since the processing of superimposing the graphical user interface G1 with the endoscopic image is performed by the controller 43 arranged at the first facility B1 where the surgery is being performed, the graphical user interface G1 can be superimposed with the endoscopic image without delay.

In addition, the controller 43 displays the endoscopic image superimposed with the graphical user interface G1 on the scope type display 24 of the doctor-side operating device 2. Accordingly, not only the mentor A2 but also the doctor A1 on the side of the operation can grasp the communication state, which can suppress difference in recognition of information between the mentor A2 and the doctor A1.

Further, the controller 43 displays the endoscopic image superimposed with the graphical user interface G1 on the touch panel display 61 at the first facility B1, the display 41 at the information sharing device 4, and the touch panel display 51 at the second facility B2. With this, the assistant A3 and the clinical engineer at the first facility B1 and the clinical engineer at the second facility B2 can also visually grasp the communication state.

The graphical user interface G1 including information about a communication state includes at least one of a communication delay time, an icon representing a degree of communication delay, and a color representing the degree of communication delay. With this, the mentor A2 can easily visually grasp the communication state delay from the communication delay time, the icon representing the degree of communication delay, or the color representing the degree of communication delay.

For example, the graphical user interface G1 including the information about the communication state is displayed in an area G1a in an upper region of the screen of the scope type display 34 and in the vicinity of the center area of the screen of the scope type display 34, as illustrated in FIG. 7

For example, as illustrated in FIG. 8, the graphical user interface G1 may display an indication G2 indicating the communication delay time as a numerical value.

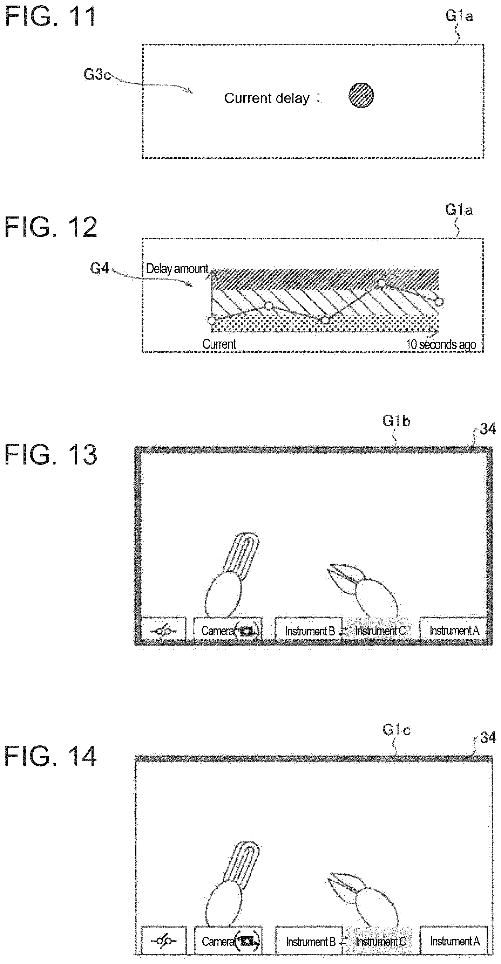

As illustrated in FIGS. 9 to 11, the color indications G3a, G3b, and G3c representing the degree of delay may include: a first color (see FIG. 9) indicating that the delay time is equal to or less than an allowable value; a second color (see FIG. 10) indicating that the delay time exceeds the allowable time by a value not more than a first predetermined value; and a third color (see FIG. 11) indicating that the delay time exceeds the allowable time by a value more than the first predetermined value. As a result, the color displayed changes in stages according to the communication delay time, so that the mentor A2 can intuitively grasp the current degree of the communication delay from the displayed color.

For example, the first color may be green or blue to indicate a low degree of delay, the second color may be yellow to indicate a medium degree of delay, and the third color may be red to indicate a high degree of delay.

Further, as illustrated in FIG. 12, the information regarding the communication state may include an indication G4 of information indicating changes over time in the delay time. With this configuration, the mentor A2 can easily grasp the tendency of the communication delay by checking the information on the change in the delay time over time. In this case, the information on the change in the delay time over time may include a graph indicating transition of the delay time. With this, the mentor A2 can easily visually grasp the transition of the communication delay with the displayed graph. Note that the graph may be displayed in different colors depending on the degree of the delay, as illustrated in FIG. 12.

Further, the color indication representing the degree of delay included in the graphical user interface G1 may be displayed in an area G1b surrounding or at the outer peripheral edge of the screen of the scope type display 34, as illustrated in FIG. 13.

Further, as illustrated in FIG. 14, the color indication representing the degree of delay included in the graphical user interface G1 may be displayed in an area G1c in the vicinity of the upper side (the upper edge) of the screen of the scope type display 34.

The controller 16 may acquire the state of communication between the mentor-side operating device 3 and the surgical robot 1 from a device that monitors the communication state of the hub 72 (82). Further, the controller 16 may be configured to acquire from the router 73 (83) the state of communication between the mentor-side operating device 3 and the surgical robot 1. Further, the controller 16 may acquire from the decoder 81 the state of communication between the mentor-side operating device 3 and the surgical robot 1.

### (Communication State Acquisition Process)

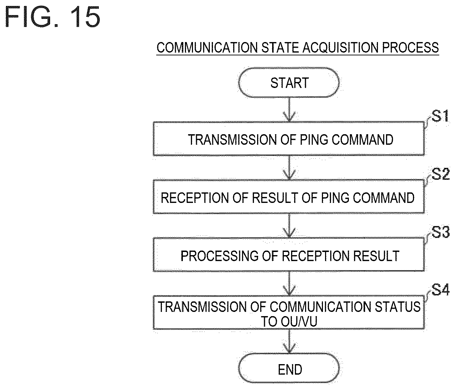

For example, the controller 16 controls the router 83 to acquire line performance information through a communication state acquisition process illustrated in FIG. 15.

In step S1 in FIG. 15, the router 83 transmits a Ping command to the router 73. When transmitting the Ping command, the following options may be set. Note that the option settings can be changed without being fixed. The option settings include, for example, a destination (the router 73), a number of repetitions, a repetition interval (in milliseconds), a content of a probing packet, and a size of the probing packet (in bytes).

In step S2, the router 73 receives the result of the Ping command. In step S3, the router 73 processes the reception result. Data obtained by processing the reception result includes, for example, a measurement date, a measurement time, an average performance value, and a packet loss value.

In step S4, the communication status is transmitted to the controller 16. Thereby, the controller 16 acquires the communication state.

### (Effects of embodiments)

One or more embodiments described above may achieve effects as described below.

In an embodiment, as described above, the controller 16 acquires the state of communication between the mentor-side operating device 3 and the surgical robot 1. The controller 43 superimposes the graphical user interface G1 including the information about the communication state acquired by the controller 16 with the endoscopic image, and displays on the scope type display 34 of the second facility B2 the graphical user interface G1 superposed with the endoscopic image. With this, even if an operation delay occurs due to the state of communication between the mentor-side operating device 3 and the surgical robot 1 that are remotely connected via the external network N, the graphical user interface G1 including the information about the communication status displayed on the scope type display 34 allows the mentor A2 to easily check the state of communication. As a result, while grasping the state of communication between the mentor-side operating device 3 and the surgical robot 1, the mentor A2 can remotely operate the surgical robot 1 from the mentor-side operating device 3 according to the communication state.

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modification) within the same.

For example, in one or more embodiments described above, the case has been described in which each of the doctor-side operating device and the mentor-side operating device includes the touch panel display and the voice communication device. However, the disclosure is not limited thereto. For example, only the mentor-side operating device may include the touch panel display and the voice communication device.

Further, in one or more embodiments described above, the case has been described in which the control process of the disclosure is performed by the plurality of controllers. However, the disclosure is not limited thereto. For example, the control process of the disclosure may be performed by one controller.

Further, in one or more embodiments described above, the case has been described in which the scope type display is provided as the first display and the second display. However, the disclosure is not limited thereto. For example, a display other than the scope type display, such as a curved panel display or a flat panel display, may be provided as the first display or the second display.

Further, in one or more embodiments described above, the case has been described in which each of the doctor-side operating device and the mentor-side operating device is provided with the switching device for switching the operation authority for operating the surgical robot. However, the disclosure is not limited to this. For example, only one of the doctor-side operating device and the mentor-side operating device may be provided with a switching device for switching the operation authority for operating the surgical robot.

Further, in one or more embodiments described above, the case has been described in which the communication status is notified by displaying the graphical user interface G1 including the information about the communication status. However, the disclosure is not limited thereto. For example, in addition to displaying the graphical user interface G1 including the information about the communication state, the voice communication device 36 may be used to notify the communication state by sound or voice message. For example, when notifying the communication state by sound, the degree of the communication delay may be notified by graduated sounds in stages such as "beep", "beep, beep", and "beep, beep, beep". When notifying the communication state by a voice message, the communication state may be notified by playing a message such as "the line delay is increasing." With such configurations, it is possible to notify the mentor A2 and also the clinical engineer A4 around the mentor A2 of the communication state. Further, the voice communication device 26 and the voice communication device 42 may be used to notify the doctor A1 or the assistant A3 of the communication status by sound or voice message. Furthermore, in addition to displaying the graphical user interface G1 including the information about the communication status, the operating handle 31 or 21 may be vibrated to notify the communication status. When notifying by such a vibration, the degree of the communication delay can be notified by the magnitude of vibration in stages.

Further, in one or more embodiments described above, the case has been described in which the voice communication device is provided in the information sharing device in order to perform the voice communication. However, the disclosure is not limited thereto. For example, the surgical robot may be provided with a voice communication device in order to perform voice communication.

Further, in one or more embodiments described above, the case has been described in which the mentor-side operating device 3 is provided in the second facility B2. However, the disclosure is not limited thereto. In the disclosure, a remote surgery support system may be configured such that a doctor-side operating device and a surgical robot are arranged in a second facility B2, the doctor-side operating device of the second facility B2 is configured, when a normal mode is selected from the mode selection, to operate the surgical robot of the second facility B2, and the doctor-side operating device of the second facility B2 is configured, when a remote mode is selected from the mode selection, to operate a surgical robot of the first facility B1 as a mentor-side operating device.

### (Aspects)

It will be appreciated by those skilled in the art that exemplary embodiments described above are specific examples of the following aspects.

### (Item 1)

A remote surgery support system including:
a surgical system located at a first facility and including a first manipulator arm holding a first surgical instrument, a second manipulator arm holding a second surgical instrument, a third manipulator arm holding an endoscope, and a doctor-side operating device configured to be operated by a doctor to operate the first to third manipulator arms,
a mentor-side operating device placed in a second facility different from the first facility and configured to be operated by a mentor to operate the surgical robot via an external network, and
one or more controllers, wherein
the doctor-side operating device includes a first display configured to display an endoscopic image acquired by the endoscope,
the mentor-side operating device includes a second display configured to display the endoscopic image transmitted via the external network, and
the one or more controllers is configured to acquire a state of communication between the mentor-side operating device and the surgical system and display, on the second display, a graphical user interface including information about the communication state with the graphical user interface being superimposed with the endoscopic image.

### (Item 2)

The remote surgery support system according to Item 1, wherein
the graphical user interface including the information about the communication state includes at least one of a communication delay time, an icon representing a degree of communication delay, and a color representing the degree of communication delay.

### (Item 3)

The remote surgery support system according to Item 2, wherein
the color representing the degree of communication delay comprises a first color indicating that a delay time is equal to or less than an allowable value, a second color indicating that the delay time exceeds the allowable value by a value not more than a first predetermined value, and a third color indicating that the delay time exceeds the allowable value by a value more than the first predetermined value.

### (Item 4)

The remote surgery support system according to any one of Items 1 to 3, wherein
the information about the communication state includes information indicating a change in the delay time over time.

(Item 5) The remote surgery support system according to Item 4, wherein
the information indicating changes in the delay time over time includes a graph indicating transition of the delay time.

### (Item 6)

The remote surgery support system according to any one of Items 1 to 5, wherein
the one or more controllers is provided at the first facility, and configured to display, on the second display via the external network, the endoscopic image superimposed with the graphical user interface.

### (Item 7)

The remote surgery support system according to Item 6, wherein
the one or more controllers is configured to display, on the first display, the endoscopic image superimposed with the graphical user interface.

### (Item 8)

The remote surgery support system of any one of Items 1 to 7, wherein
at least one of the doctor-side operating device and the mentor-side operating device comprises a switching device configured to switch an operation authority for operating the first to third manipulator arms between the doctor-side operating device and the mentor-side operating device.

### (Item 9)

The remote surgery support system according to any one of Items 1 to 8, further including:
a third display provided at the second facility and configured to display the endoscopic image acquired by the endoscope and transmitted via the external network, wherein
each of the first display and the second display is an immersive scope type display, and the third display is one of a flat panel display and a curved panel display.

### (Item 10)

The remote surgery support system according to Item 9, wherein
the doctor-side operating device includes a first voice communication device,
the mentor-side operating device includes a second voice communication device and a third voice communication device each of which is configured to perform voice communication with the first voice communication device,
the third voice communication device is provided at a position closer to the third display than the second voice communication device is, and
the second voice communication device is provided at a position closer to the second display than the third voice communication device is.

### (Item 11)

The remote surgery support system according to Item 10, wherein
the controller is configured to notify at least the second voice communication device of a sound or voice message corresponding to the communication state.

### (Item 12)

The remote surgery support system according to any one of Items 9 to 11, wherein
the one or more controllers is configured to display on the third display the endoscopic image superimposed with the graphical user interface including the information about the communication state.

### (Item 13)

The remote surgery support system according to any one of Items 9 to 12, wherein
the third display comprises a touch panel display configured to display the endoscopic image transmitted via the external network and to receive an instruction input for generating an instructional image, and
the one or more controllers is configured to display on the first display the instructional image based on the instruction input received by the touch panel display with the instructional image being superimposed on the endoscopic image.

### (Item 14)

The remote surgery support system according to any one of Items 1 to 13, wherein
the doctor-side operating device includes first operating handles configured to operate the first to third manipulator arms,
the mentor-side operating device includes second operating handles configured to be operated by the mentor to operate the first to third manipulator arms, and
the one or more controllers is configured, based on the communication state, to notify the communication state by vibrating at least one of the second operating handles.

### (Item 15)

The remote surgery support system according to any one of Items 1 to 14, wherein
the surgical system includes a surgical robot that supports the first to third manipulator arms.

The invention includes other embodiments or modifications in addition to one or more embodiments and modifications described above without departing from the spirit of the invention. The one or more embodiments and modifications described herein are to be considered in all respects as illustrative, and not restrictive. The scope of the invention is indicated by the appended claims rather than by the foregoing description. Hence, all configurations including the meaning and range within equivalent arrangements of the claims are intended to be embraced in the invention.

## Claims

1. A remote surgery support system comprising:
a surgical system located at a first facility and including a first manipulator arm holding a first surgical instrument, a second manipulator arm holding a second surgical instrument, a third manipulator arm holding an endoscope, and a doctor-side operating device configured to be operated by a doctor to operate the first to third manipulator arms;
a mentor-side operating device located in a second facility different from the first facility and configured to be operated by a mentor to operate via an external network the first to third manipulator arms; and
one or more controllers, wherein
the doctor-side operating device includes a first display configured to display an endoscopic image acquired by the endoscope,
the mentor-side operating device includes a second display configured to display the endoscopic image transmitted via the external network, and
the one or more controllers is configured to acquire a state of communication between the mentor-side operating device and the surgical system and display, on the second display, a graphical user interface including information about the communication state with the graphical user interface being superimposed with the endoscopic image.

2. The remote surgery support system according to claim 1, wherein
the graphical user interface including the information about the communication state includes at least one of a communication delay time, an icon representing a degree of communication delay, and a color representing the degree of communication delay.

3. The remote surgery support system according to claim 2, wherein
the color representing the degree of communication delay comprises a first color indicating that a delay time is equal to or less than an allowable value, a second color indicating that the delay time exceeds the allowable value by a value not more than a first predetermined value, and a third color indicating that the delay time exceeds the allowable value by a value more than the first predetermined value.

4. The remote surgery support system according to any one of claims 1 to 3, wherein
the information about the communication state includes information indicating changes in a delay time over time.

5. The remote surgery support system according to claim 4, wherein
the information indicating changes in the delay time over time includes a graph indicating transition of the delay time over time.

6. The remote surgery support system according to any one of claims 1 to 5, wherein
the one or more controllers is provided at the first facility, and configured to display, on the second display via the external network, the endoscopic image superimposed with the graphical user interface.

7. The remote surgery support system according to claim 6, wherein
the one or more controllers is configured to display, on the first display, the endoscopic image superimposed with the graphical user interface.

8. The remote surgery support system according to any one of claims 1 to 7, wherein
at least one of the doctor-side operating device and the mentor-side operating device comprises a switching device configured to switch an operation authority for operating the first to third manipulator arms between the doctor-side operating device and the mentor-side operating device.

9. The remote surgery support system according to any one of claims 1 to 8, further comprising:
a third display provided at the second facility and configured to display the endoscopic image acquired by the endoscope and transmitted via the external network, wherein
each of the first display and the second display is an immersive scope type display, and the third display is one of a flat panel display and a curved panel display.

10. The remote surgery support system according to claim 9, wherein
the doctor-side operating device includes a first voice communication device,
the mentor-side operating device includes a second voice communication device and a third voice communication device each of which is configured to perform voice communication with the first voice communication device,
the third voice communication device is provided at a position closer to the third display than the second voice communication device is, and
the second voice communication device is provided at a position closer to the second display than the third voice communication device is.

11. The remote surgery support system according to claim 10, wherein
the controller is configured to notify at least the second voice communication device of a sound or voice message corresponding to the communication state.

12. The remote surgery support system according to any one of claims 9 to 11, wherein
the one or more controllers is configured to display on the third display the endoscopic image superimposed with the graphical user interface including the information about the communication state.

13. The remote surgery support system according to any one of claims 9 to 12, wherein
the third display comprises a touch panel display configured to display the endoscopic image transmitted via the external network and to receive an instruction input for generating an instructional image, and
the one or more controllers is configured to display on the first display the instructional image based on the instruction input received by the touch panel display with the instructional image being superimposed on the endoscopic image.

14. The remote surgery support system according to any one of claims 1 to 13, wherein
the doctor-side operating device includes first operating handles configured to operate the first to third manipulator arms,
the mentor-side operating device includes second operating handles configured to be operated by the mentor to operate the first to third manipulator arms, and
the one or more controllers is configured, based on the communication state, to notify the communication state by vibrating at least one of the second operating handles.

15. The remote surgery support system according to any one of claims 1 to 14, wherein
the surgical system includes a surgical robot that supports the first to third manipulator arms.
